# EUROPÄISCHE PATENTANMELDUNG

(11) **EP 0 678 293 A2**
(43) Veröffentlichungstag der Anmeldung: **25.10.1995**
(21) Anmeldenummer: 95103704.3
(22) Anmeldetag: 15.03.1995
(51) Int. Cl.: A61K 7/48, A61K 7/06

(54) **Pyridoxinester enthaltendes Präparat, Verfahren zu dessen Herstellung und dessen Verwendung**

(30) Priorität: 25.03.1994 CH 914/94
(71) Anmelder: F. HOFFMANN-LA ROCHE AG, CH-4002 Basel (CH)
(72) Erfinder: Karge, Reinhard, D-79219 Staufen (DE); Pauling, Horst, CH-4103 Bottmingen (CH)
(74) Vertreter: Kellenberger, Marcus, Dr.

(57) **Zusammenfassung**

Die vorliegende Erfindung betrifft ein topisch applizierbares Mittel, welches mindestens eine lipophile Phase sowie eine physiologisch wirksame Menge Pyridoxin enthält. Das Pyridoxn liegt als Pyridoxintripropionat vor. Die Menge an Pyridoxintripropionat beträgt 0,05-30 Gew% der Gesamtmenge des Mittels.

## Beschreibung

Die vorliegende Erfindung betrifft ein topisch applizierbares Mittel und zwar ein Pyridoxintripropionat enthaltendes Mittel sowie ein Verfahren zu dessen Herstellung und dessen Verwendung.

Pyridoxin-hydrochlorid gehört zu der Gruppe der wasserlöslichen Vitamine. In unpolaren Flüssigkeiten ist Pyridoxin-hydrochlorid praktisch unlöslich. Die lipophoben Eigenschaften von Pyridoxin-hydrochlorid schränken seine Anwendung in kosmetischen und dermatologischen Präparaten stark ein. Insbesondere ist es nicht möglich, Pyridoxin-hydrochlorid in Hautölen, Salben und Cremes sowie in anderen topisch applizierbaren Mitteln in nennenswerten Konzentrationen einzusetzen. Dies wäre jedoch wegen der positiven Wirkung des Pyridoxins für die Haut z.B. in bezug auf die Wundheilung oder die Reduktion der Sebumproduktion, sowie wegen der positiven Wirkung des Pyridoxins auf die Verstärkung der UV-Absorption in Cremes wünschenswert.

Eine Möglichkeit, die Fettlöslichkeit des Pyridoxin-hydrochlorids zu erhöhen, besteht in der teilweisen oder vollständigen Veresterung der drei im Molekül vorhandenen Hydroxygruppen mit aliphatischen Carbonsäuren.

Eine Reihe von Publikationen befasst sich mit der Herstellung und den Eigenschaften von Pyridoxin-di-und-triestern. Für alle beschriebenen Pyridoxinester wird die volle Vitamin-B₆-Aktivität angegeben. (T. Sakuragi, F.A. Kummerow, J.Am. Oil Chem. Soc. 33 1956, 116; T. Sakuragi, F.A. Kummerow, J.Am. Chem. Soc. 78 1956, 839; Deutsche Patentschrift 741154).

In der deutschen Patentschrift 741154 wird die Acylierung von Pyridoxin-hydrochlorid, beispielsweise die Herstellung von Pyridoxintripropionat, beschrieben. In dieser Patentschrift wird festgehalten, dass die gewonnenen Acylverbindungen im Gegensatz zum nicht acylierten Vitamin B₆ in Fetten, Oelen und in Fettlösungsmitteln wie z.B. Chloroform leicht löslich seien. Diese Aussage wird jedoch nicht durch ein Ausführungsbeispiel oder durch Angaben über die Höhe der Löslichkeit belegt.

Die Gruppe um T. Sakuragi (T. Sakuragi, F.A. Kummerow, J.Am Oil Chem. Soc. 33 1956, 116) hat später festgestellt, dass diese Aussage nicht zutrifft. Hinsichtlich der Löslichkeit wird in T. Sakuragi, F.A. Kummerow, J.Am Oil Chem. Soc. 33 1956, 116 auf die schlechte Fettlöslichkeit der kurzkettigen Pyridoxinester (Pyridoxintriacetat und Pyridoxintripropionat ) im Vergleich zu Pyridoxinestern längerkettiger Carbonsäuren hingewiesen. Es wird festgestellt, dass die kurzkettigen Pyridoxinester beinahe unlöslich in Fetten seien. Pyridoxinester längerkettiger Carbonsäuren, beispielsweise Pyridoxinoctanoat, lösen sich bis zu etwa 1% in Pflanzenölen. Für Pyridoxintripalmitat wird eine Löslichkeit von 0,1% bei 3^{o} C in Olivenöl angegeben. Im Rahmen der vorliegenden Erfindung wurde nun gefunden, dass Pyridoxintripropionat in Pflanzenöl überraschenderweise bis zu 50 Gew.% löslich ist.

Ein Pyridoxintripalmitat enthaltendes kosmetisches Präparat wird beispielsweise in der europäischen Patentanmeldung EP-0 001 079 beschrieben. Der Einsatz von Pyridoxintripalmitat hat den Nachteil, dass aufgrund des Verhältnises vom Molekulargewicht der Tripalmitatreste zum Molekulargewicht des Pyridoxins der Anteil an Pyridoxin in Pyridoxintripalmitat sehr gering ist. Somit ist es nicht möglich durch den Einsatz von Pyridoxintripalmitat, Pyridoxin als physiologisch wirksame Komponente in höheren Konzentrationen in topisch applizierbaren Mitteln zur Verfügung zu stellen.

Der vorliegenden Erfindung liegt nun die Aufgabe zugrunde, topisch applizierbare Mittel auf Fettbasis oder auf der Basis nicht wässriger Grundstoffe mit physiologisch wirksamen Pyridoxinkonzentrationen über einen weiten Konzentrationsbereich zur Verfügung zu stellen.

Das erfindungsgemässe Mittel zur Lösung dieser Aufgabe enthält mindestens eine lipophile Phase und ist dadurch gekennzeichnet, dass das Pyridoxin als Pyridoxintripropionat vorliegt.

Mit der Verbindung Pyridoxintripropionat können Pyridoxinkonzentrationen in lipophilen Phasen von mehr als 50% erzielt werden.

Der Ausdruck "lipophile Phase" bedeutet im Rahmen der vorliegenden Erfindung Grundstoffe ölhaltiger Einphasensysteme als Basis für Hautöle und Hautfette oder die Fettphase einer Emulsion. Als nicht wässrige Grundstoffe für Hautöle, Hautfette und für die Fettphase einer Emulsion kommen in Frage pflanzliche und mineralische Oele, Siliconöle, Paraffinöle, Paraffin, Vaselin, flüssige Wachse, Fette, Fettsäureester, Fettalkohole, Lecithine, ölige Auszüge verschiedener Pflanzen und dergleichen. Weitere Bestandteile der Fettphase sind Emulgatoren wie Wollwachs, Wollwachsalkohole, höhere aliphatische Alkohole wie Cetylalkohol und Stearylalkohol, Seifen ein-,zwei und dreiwertiger Kationen, Ester polyvalenter Alkohole, Konsistenzregler, öllösliche Konservierungsmittel und dergleichen.

Die erfindungsgemässen topisch applizierbaren Mittel enthalten Pyridoxintripropionat zweckmässigerweise in Gehalten zwischen 0,05-30 Gew%, sodass bei äusserer Anwendung genug Wirkstoff zur Aufnahme durch die Haut zur Verfügung steht. Vorzugsweise beträgt der Gehalt an Pyridoxintripropionat 0,5-20 Gew%. Pyridoxintripropionat wird während oder nach der Aufnahme durch die Haut durch die Einwirkung des menschlichen oder tierischen Organismus in Pyridoxin umgewandelt.

Propionsäure, die bei der Spaltung des Tripropionats beispielsweise durch den Einfluss der Hautfeuchtigkeit freigesetzt wird, ist eine toxikologisch unbedenkliche Verbindung. Propionsäure ist als Konservierungsmittel in Lebensmitteln zugelassen.

Propionsäure hat im Unterschied zur nächst höheren homologen Verbindung, der Buttersäure, einen angenehmen Geruch.

Pyridoxintripropionat besitzt bei unmittelbarer Anwendung in Salben, Cremes und anderen für die Behandlung der Haut geeigneten Zubereitungen volle Vitamin B₆ Wirksamkeit.

Die erfindungsgemässen topisch applizierbaren Mittel können zusätzlich zu Pyridoxintripropionat alle für Haut und Haar physiologisch verträglichen Wirk- und/oder Zusatzstoffe enthalten.

Solche Wirkstoffe sind beispielsweise die zur Haut- und Haarpflege bereits eingesetzten Vitamine A und F oder Panthenol; Aminosäuren wie Methionin, Cystin, Cystein, Alanin und dergleichen.

Weitere an sich bekannte Wirkstoffe sind Pflanzenextrakte, durchblutungsfördernde und entzündungshemmende Stoffe, Lichtfilterwirkstoffe und dergleichen.

Gebräuchliche Zusatzstoffe sind beispielsweise Farbstoffe, Parfumöle, Dispersionsmittel, Emulgatoren, Verdickungsmittel, Konservierungsmittel, Stabilisatoren und dergleichen.

Die erfindungsgemässen topisch applizierbaren Mittel können sowohl als Kosmetika für Haut und/oder Haar als auch als pharmazeutische Präparate eingesetzt werden. Als kosmetische bzw. dermatologische Grundlage kann jede übliche Zubereitung dienen, die den kosmetischen bzw. dermatologischen Anforderungen entspricht z.B. Körperöle, Salben, Cremes, Lotionen, Gele, Shampoos, Sprays, Stifte, Lippenstifte, Gesichtswasser und dergleichen.

Zur Herstellung der erfindungsgemässen topisch applizierbaren Mittel wird Pyridoxintripropionat mit einer der oben beschriebenen lipophilen Phasen eventuell mit einem oder mehreren anderen Wirk - und/oder Zusatzstoffen vermischt. Das Pyridoxintripropionat ist in Pflanzenöl überraschenderweise bis zu 50 Gew.% löslich. Die Herstellung der erfindungsgemässen topisch applizierbaren Mittel ist dem Fachmann geläufig und in den verschiedenen einschlägigen Handbüchern ausführlich beschrieben. Es wird hierzu beispielhaft auf K. Schrader "Grundlagen und Rezepturen der Kosmetika" 1989, Dr. Alfred Hüthig Verlag Heidelberg hingewiesen.

Pyridoxintripropionat kann hergestellt werden, indem man Pyridoxinhyrochlorid mit Propionsäure, Propionsäureanhyrid oder Propionsäurechlorid verestert. Die Umsetzung kann unter an sich bekannten Veresterungsbedingungen durchgeführt werden. Die Veresterung wird zweckmässigerweise mit einem 2-3 fachen Ueberschuss an Veresterungsreagenz in Gegenwart einer Base durchgeführt. Eine geeignete Base kann Triethylamin oder Natriumpropionat sein. Die Veresterung erfolgt zweckmässigerweise in einem Temperaturbereich zwischen Raumtemperatur und der Siedetemperatur des Reaktionsgemisches, vorzugsweise bei der letztgenannten Temperatur. Das Rohprodukt kann entweder extraktiv aufgearbeitet oder durch fraktionierte Destillation gereinigt werden.

Die erfindungsgemässen Mittel werden verwendet, indem man auf die zu behandelnde Haut oder Kopfhaut eine wirksame Menge des Mittels appliziert. Diese Applikation kann in üblicher Weise erfolgen z.B. durch Einreiben oder Besprühen.

Die Erfindung wird durch die nachfolgenden Beispiele veranschaulicht, ohne auf diese beschränkt zu sein.

### I. Herstellung einer 50% igen Lösung von Pyridoxintripropionat in Pflanzenöl.

50 Gew% Pyridoxintripropionat werden vorgelegt und in 50 Gew% Pflanzenöl unter starkem Rühren gelöst. Es entsteht eine homogene Lösung.

Zur Herstellung von Pyridoxintripropionat wird 20.6 g ( 100 mMol) Pyridoxinhydrochlorid in 200 ml Hexan suspendiert. Unter Rühren bei Raumtemperatur wird nacheinander 20.2 g (200 mMol) Triethylamin und 52.1 g (400 mMol) Propionsäureanhydrid zugegeben. Die Suspension wird innerhalb von 20 Minuten zum Sieden erhitzt und 12 Stunden unter Rückfluss gekocht. Nach circa 12 Stunden Reaktionszeit lässt man die Suspension abkühlen. Die flüssige Phase wird vom feinen kristallinen Feststoff mit Hilfe einer Nutsche getrennt. Der Rückstand wird auf der Nutsche zweimal mit je 10 ml Hexan gewaschen. Der Rückstand besteht aus Triethylaminhydrochlorid. Die organische Phase, die das Reaktionsprodukt enthält, wird am Rotationsverdampfer eingeengt. Dabei destilliert der grösste Teil des Triethylamins über. Anschliessend wird die Propionsäure/Propionsäureanhydrid Mischung unter Wasserstrahlvakuum abdestilliert. Das gelb bis rotbraun gefärbte Rohprodukt wird unter Oelpumpenvakuum destilliert. Man erhält 31.2 g Pyridoxintripropionat als farblose bis schwach gelbe Flüssigkeit mit einem Siedepunkt von Kp _{0.3} = 160 ^{o} C. Die Ausbeute beträgt 92.5 % d. Th.

### II Rezepturen für erfindungsgemäss zusammengesetzte topisch applizierbare Mittel.

| IIa Körper-/ Gesichtsöl | |
|---|---|
| Bestandteile | Gewichtsprozent |
| Pyridoxintripropionat | 30 |
| Isopropylpalmitat | 70 |

| IIb-1 Creme | |
|---|---|
| Bestandteile | Gewichtsprozent |
| Pyridoxintripropionat | 15.0 |
| Stearinsäure | 2.5 |
| Polyethylenglykolether von Oleylalkohol | 2.0 |
| Glyceryl-Monostearat | 3.5 |
| Isopropylpalmitat | 7.5 |
| Polyethylenglykolether von Lanolinalkohol | 2.0 |
| Bienenwachs | 1.5 |
| Propylparaben | 0.1 |
| Methylparaben | 0.1 |
| Triethanolamin | 1.0 |
| Propylenglykcol | 3.5 |
| Panthenol | 1.0 |
| Wasser | 45.0 |
| Vaseline | ad 100 |

| IIb-2 Creme | |
|---|---|
| Bestandteile | Gewichtsprozent |
| Pyridoxintripropionat | 5.0 |
| Labrafil M 1944 CS | 3.0 |
| Tefose 63 | 25.0 |
| Glycerin rein | 10.0 |
| 2-Phenoxyethanol | 0.5 |
| Wasser | 56.5 |

| IIb-3 Creme | |
|---|---|
| Bestandteile | Gewichtsprozent |
| Pyridoxintripropionat | 1.0 |
| Labrafil M 1944 CS | 3.0 |
| Tefose 63 | 25.0 |
| Glycerin rein | 10.0 |
| 2-Phenoxyethanol | 0.5 |
| Wasser | 60.5 |

Bei den Markennamen Labrafil M 1944 CS und Tefose 63 handelt es sich um folgende Produkte:
Tefose 63 ist ein Ethylenglycol und Polyoxyethylenglycol Palmito-Stearat.

Labrafil M 1944 CS ist ein ungesättigtes polyglykolisiertes Glycerid hergestellt durch eine Alkoholyse von Aprikosenkernöl und konstituiert aus Glyceriden und Polyethylenglykolester.

| IIc Lippenstift | |
|---|---|
| Bestandteile | Gewichtsprozent |
| Pyridoxintripropionat | 5.0 |
| Candelillawachs | 12.5 |
| Bienenwachs | 11.5 |
| Cetylalkohol | 5.0 |
| Petrolatum | 15.0 |
| Butylstearat | 6.0 |
| zugelassene Farbstoffe | 7.5 |
| Rosenöl | 0.5 |
| Rizinusöl | ad 100 |

| IId Lotion | |
|---|---|
| Bestandteile | Gewichtsprozent |
| Pyridoxintripropionat | 1.0 |
| Stearinsäure | 5.0 |
| Glycerylstearat und Polyethylenglykol(100)-stearat | 4.5 |
| Isopropylpalmitat | 1.0 |
| Mineralöl | 1.0 |
| Propylparaben | 0.1 |
| Methylparaben | 0.2 |
| Propylenglykol | 3.0 |
| Wasser | ad 100 |

| IIe Shampoo | |
|---|---|
| Bestandteile | Gewichtsprozent |
| Pyridoxintripropionat | 1.5 |
| Ammoniumlaurylsulfat | 25.0 |
| Cocoamidopropyl-Betain | 5.0 |
| Lauramide DEA ( Gemisch von Ethanolamiden der Laurinsäure ) | 2.0 |
| Propylparaben | 0.1 |
| Methylparaben | 0.15 |
| Wasser | ad 100 |

| IIf Gesichtswasser | |
|---|---|
| Bestandteile | Gewichtsprozent |
| Pyridoxintripropionat | 3.0 |
| Milchsäure | 0.2 |
| Allantoin-N-acetyl-DL-methionat | 1.0 |
| Methanol | 0.1 |
| Ethanol | 45.0 |
| Wasser | ad 100 |

## Patentansprüche

1. Topisch applizierbares Mittel, welches mindestens eine lipophile Phase sowie eine wirksame Menge Pyridoxin enthält, dadurch gekennzeichnet, dass das Pyridoxin als Pyridoxintripropionat vorliegt.

2. Topisch applizierbares Mittel gemäss Anspruch 1, dadurch gekennzeichnet, dass die Menge an Pyridoxintripropionat 0,05-30 Gew% der Gesamtmenge des Mittels beträgt, vorzugsweise 0,5-20 Gew%.

3. Topisch applizierbares Mittel gemäss Anspruch 1 oder 2, dadurch gekennzeichnet, dass neben Pyridoxin ein oder mehrere weitere Wirk- und/oder Zusatzstoffe enthalten sind.

4. Topisch applizierbares Mittel gemäss einem der Ansprüche 1-3, dadurch gekennzeichnet, dass es in Form eines Körperöls, einer Creme, einer Lotion, eines Shampoos, eines Gesichtswassers oder eines Lippenstifts vorliegt.

5. Verfahren zur Herstellung eines topisch applizierbaren Mittels gemäss Anspruch 1, dadurch gekennzeichnet, dass man eine wirksame Menge Pyridoxintripropionat in einer lipophilen Phase löst, gegebenenfalls in Gegenwart eines oder mehrer weiterer Wirkstoffe und/oder Zusatzstoffe.

6. Verfahren zur Pflege der Haut oder des Haares, dadurch gekennzeichnet, dass man eine wirksame Menge eines in Anspruch 1 genannten Mittels appliziert.

7. Topisch applizierbares Mittel gemäss Anspruch 1 zur Verwendung in einem Verfahren zur therapeutischen Behandlung von Haut und/ oder Haar des menschlichen oder tierischen Körpers.
